# DEMANDE DE BREVET EUROPEEN

(11) **EP 1 232 836 A1**
(43) Date de publication de la demande: **21.08.2002**
(21) Numéro de dépôt: 01103935.1
(22) Date de dépôt: 19.02.2001
(51) Int. Cl.: B25B 9/02, A61B 17/30, B25J 7/00

(54) **Porte-brucelles**

(71) Demandeur: Manufactures d'Outils Dumont S.A., 2924 Montignez (CH)
(72) Inventeur: Sgobero, Christian, 2924 Montignez (CH); Sgobero, Richard, 2924 Montignez (CH)
(74) Mandataire: KIRKER & Cie S.A.

(57) **Abrégé**

Préhenseur pour brucelles comportant un boîtier (1), destiné à être monté sur le bras d'un robot, renfermant d'une part un dispositif de fixation et d'indexation (6-14) d'une brucelle (3) et d'autre part un dispositif d'actionnement des branches de la brucelle. Ce dispositif d'actionnement comporte un organe moteur (4,17) actionnant des poussoirs (21) agissant sur les branches de ladite brucelle.

## Description

De plus en plus les opérations de manutention, de positionnement, d'une manière générale toutes les opérations manuelles tendent à s'automatiser et à se robotiser. Dans de nombreuses applications, horlogères, médicales, de recherche, etc. ces opérations manuelles sont effectuées à l'aide de brucelles. Pour pouvoir automatiser ou robotiser de telles opérations manuelles utilisant des brucelles il faut, à l'instar de nombreux outils qui disposent de porte-outils, disposer de porte-brucelles permettant de faire le lien mécanique entre un robot et une ou plusieurs brucelles et permettant également l'actionnement par le robot de ces brucelles.

La présente invention a pour but la réalisation d'un préhenseur ou porte-brucelles destiné à être monté sur l'organe mobile d'un robot et capable de recevoir une paire de brucelles et d'actionner celle-ci pour la préhension, l'orientation, le transport et le posage d'objets devant être manipulés avec des brucelles. Un tel préhenseur constitue en fait une interface mécanique entre un robot et une paire de brucelles qui permette ainsi d'automatiser et de robotiser toutes sortes d'opérations réalisées actuellement manuellement avec des brucelles.

La présente invention a pour objet un préhenseur ou porte-brucelles destiné à être monté sur un organe mobile d'un robot et adapté à recevoir des brucelles et à les actionner, qui se distingue par les caractéristiques énumérées à la revendication 1.

Le dessin annexé illustre schématiquement et à titre d'exemple une forme de porte-brucelles selon l'invention.

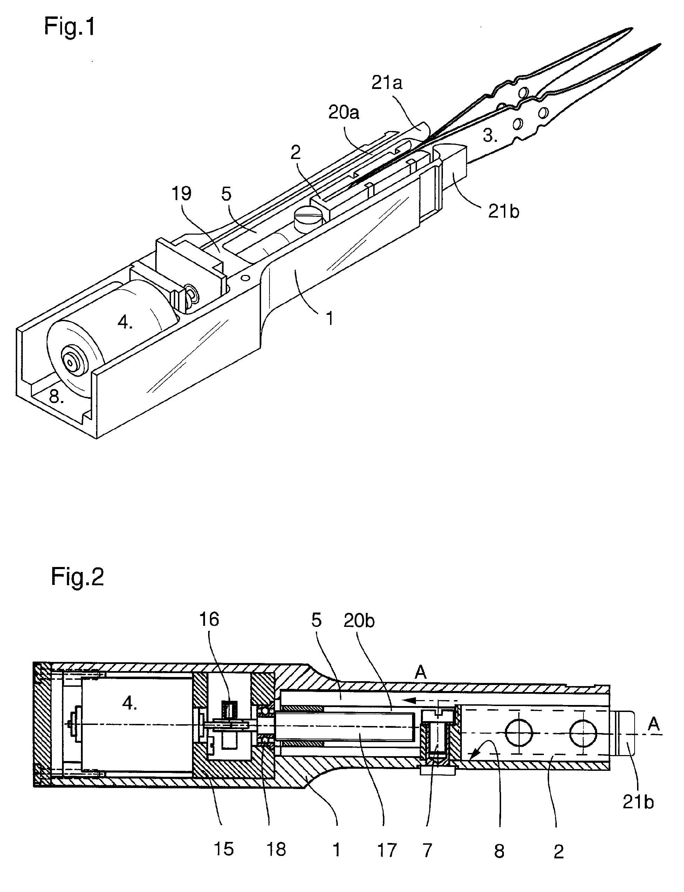

La figure 1 est une vue en perspective du porte-brucelles.
La figure 2 est une coupe longitudinale du porte-brucelles.

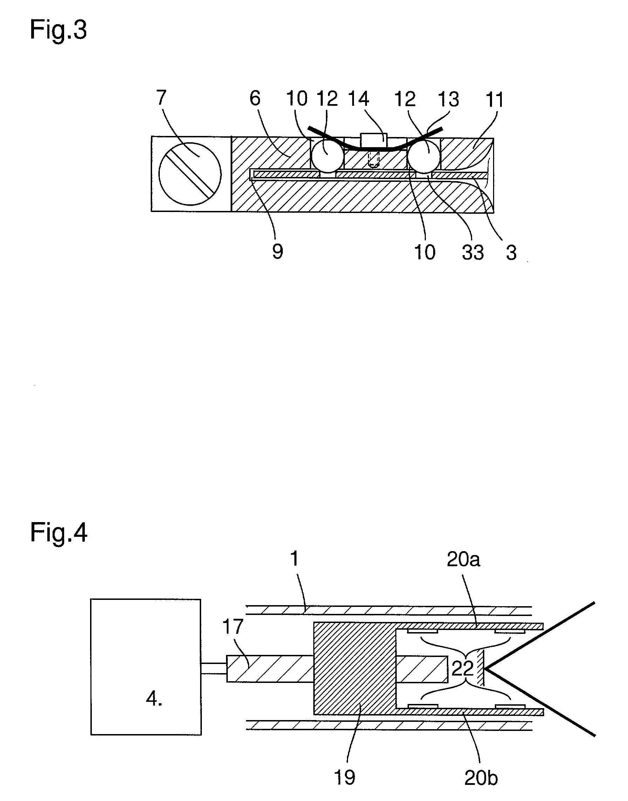

La figure 3 illustre en coupe suivant la ligne A-A de la figure 2 le dispositif de fixation d'une brucelle dans le porte-brucelles.
La figure 4 est un schéma de principe du fonctionnement du dispositif d'actionnement des brucelles.
Les figures 5 et 5a illustrent un magasin recevant des brucelles devant être prises par le porte-brucelles.
La figure 6 illustre une brucelle prévue pour son utilisation avec le porte-brucelles.

Le préhenseur ou porte-brucelles objet de la présente invention est destiné à être monté sur le bras d'un robot et à recevoir différentes brucelles selon l'objet à saisir, ainsi qu'à actionner ces brucelles pour la préhension et la pose dudit objet.

Ce préhenseur comporte un bâti ou boîtier 1 comportant des moyens de fixation au bras d'un robot et renfermant un dispositif de fixation ou d'indexage 2 d'une brucelle 3 et un dispositif d'actionnement de cette brucelle comportant un moteur 4 commandant les mouvements d'une pince 5 agissant sur les branches de la brucelle.

Dans l'exemple illustré le dispositif de fixation ou d'indexage 2 de la brucelle est constitué par une pièce 6 fixée à l'aide d'une vis 7 sur le fond 8 du boîtier 1. La pièce 6 présente une fente 9 disposée parallèlement à l'axe longitudinal du bâti 1 et ouverte en direction de l'extrémité avant, ouverte, du bâti 1. Les dimensions, longueur, largeur et épaisseur de cette fente 9 sont adaptées aux dimensions de la partie arrière des brucelles 3 devant être fixées dans le préhenseur.

Ce dispositif d'indexage comporte encore deux perçages 10 traversant l'une 11 des ailes de la pièce 6 et dans lesquels sont logées des billes 12 soumises à l'action d'un ressort 13 à lame fixé sur la pièce 6 à l'aide d'une vis 14. Lorsqu'il n'y a pas de brucelles dans le dispositif d'indexage, les billes 12 sont appliquées contre la face interne de l'autre aile de la pièce 6 sous l'effet du ressort 13.

Les brucelles destinées à être fixées dans le préhenseur comportent sur leur partie arrière, où les branches sont reliées ensemble, deux trous dont l'entraxe correspond à l'entraxe entre les perçages 10 de l'aile 11 de la pièce 6.

Lors de l'introduction d'une brucelle par sa partie arrière dans le dispositif d'indexage, les billes 12 sont repoussées puis tombent dans les trous de la brucelle et maintiennent celle-ci en position de service. Les billes 12 coopérant avec les trous de la brucelle positionnent exactement celle-ci dans le préhenseur et la maintiennent avec une force suffisante pour pouvoir effectuer avec les brucelles les opérations prévues.

Le dispositif d'indexage décrit et illustré n'est qu'une exécution parmi de nombreuses possibles. Ce dispositif d'indexage ne nécessite aucune action extérieure mais il est évident que les organes de positionnement, billes 12 et trous correspondants des brucelles, pourraient être différents pour autant qu'ils permettent un positionnement précis et une interchangeabilité des brucelles. Les organes de positionnement correspondants peuvent être réalisés de toute autre manière connue sans sortir du cadre de l'invention.

De même, on peut prévoir des dispositifs de fixation et d'indexage commandés par une action extérieure, le pincement de l'arrière de la brucelle dans la pièce 6 pouvant être commandé par l'arrivée en fin de course de positionnement de cette brucelle.

Les caractéristiques essentielles de ce dispositif de fixation et d'indexation étant le positionnement exact de la brucelle dans sa position de service par rapport au préhenseur et le fait que sa fixation soit réversible c'est-à-dire que les brucelles soient interchangeables.

Le préhenseur comporte encore un dispositif d'actionnement d'une brucelle 3 positionnée dans le dispositif de fixation et d'indexage précédemment décrit. Ce dispositif d'actionnement comporte un moteur 4 logé dans l'arrière du boîtier 1 fixé sur une entretoise 15 présentant en coupe la forme d'un U, elle-même logée dans le boîtier 1. L'axe 16 du moteur 4 traverse une aile de l'entretoise 15 et est relié par un accouplement 16 à l'extrémité arrière d'un arbre 17 dont l'extrémité arrière est pivotée à l'aide d'un roulement à bille 18 dans l'autre aile de l'entretoise 15. La partie de cet arbre 17 s'étendant parallèlement à l'axe longitudinal du boîtier 1 en direction du dispositif de fixation est filetée et coopère avec un écrou 19 formant la partie arrière d'une pince comportant deux bras 20a, 20b s'étendant à l'intérieur du boîtier 1 de part et d'autre du dispositif de fixation et se terminant chacun par un poussoir 21 a, 21 b émergeant hors du boîtier 1 et prenant chacun appui sur une des branches d'une brucelle positionnée dans le dispositif de fixation.

Ainsi, en provoquant la rotation du moteur 4 dans un sens ou dans l'autre on déplace la pince en avant ou en arrière provoquant le rapprochement des branches de la brucelle par l'action des poussoirs 21 a, 21b ou l'écartement de ses branches par leur action élastique propre, ces branches de la brucelle restant toujours en contact avec lesdits poussoirs 21a, 21b.

La forme d'exécution décrite du dispositif d'actionnement est bien entendu un exemple non limitatif. Dans d'autres formes d'exécution la liaison cinématique entre l'arbre du moteur 4 et les poussoirs 21a, 21b peut être très différente, la pince peut être remplacée par des leviers articulés, etc. De même, le moteur 4 peut être remplacé par un vérin ou un électro-aimant.

Ce qui importe c'est que l'organe moteur, moteur électrique par exemple du type pas à pas, vérin ou électro-aimant, actionne des poussoirs qui provoquent les mouvements de fermeture des branches de la brucelle et servent de butée à l'ouverture de celle-ci contre lesquelles ces branches restent en appui.

La commande de l'organe moteur 4 peut se faire électroniquement ou informatiquement en fonction du programme devant être exécuté par le robot. Mais quel que soit le mode de commande de cet organe moteur, il faut que celui-ci soit asservi à la force de serrage des brucelles pour ne pas dépasser une valeur prédéterminée par la force de serrage d'un objet entre les branches des brucelles.

Cet asservissement du moteur à la force de serrage des brucelles peut se faire à l'aide de quatre jauges de contrainte 22 fixées sur la face interne des bras 20a et 20b de la pince de serrage. Ces jauges de contrainte 22 sont montées en pont de Wheatstone de manière à délivrer une tension de mesure positive lorsque ces bras 20a, 20b fléchissent vers l'extérieur en réaction à leur appui sur les branches de la brucelle. Cette tension augmente en fonction de la fermeture de la brucelle puis de la force de serrage exercée par celle-ci sur un objet. Une comparaison de cette tension de mesure positive avec une tension de référence correspondant à la force de serrage désirée permet de délivrer un signal de commande d'arrêt du moteur 4.

Pour libérer l'objet et ouvrir la brucelle, on fait tourner le moteur dans le sens inverse, provoquant la rétraction de la pince dans le boîtier, la brucelle s'ouvre et lorsque celle-ci est complètement ouverte les bras 20a, 20b de la pince ne sont plus fléchis, la tension de mesure est égale à zéro et provoque par comparaison avec une autre tension de référence l'arrêt du moteur 4.

Pour utiliser de façon optimale un tel préhenseur de brucelles il faut que celui-ci, fixé au bras d'un robot, puisse venir automatiquement choisir dans un posage ou magasin de brucelles une brucelle déterminée, la prendre puis ultérieurement la replacer dans le magasin.

De nombreux posages ou magasins peuvent être envisagés dont un va être décrit ci-dessous à titre d'exemple.

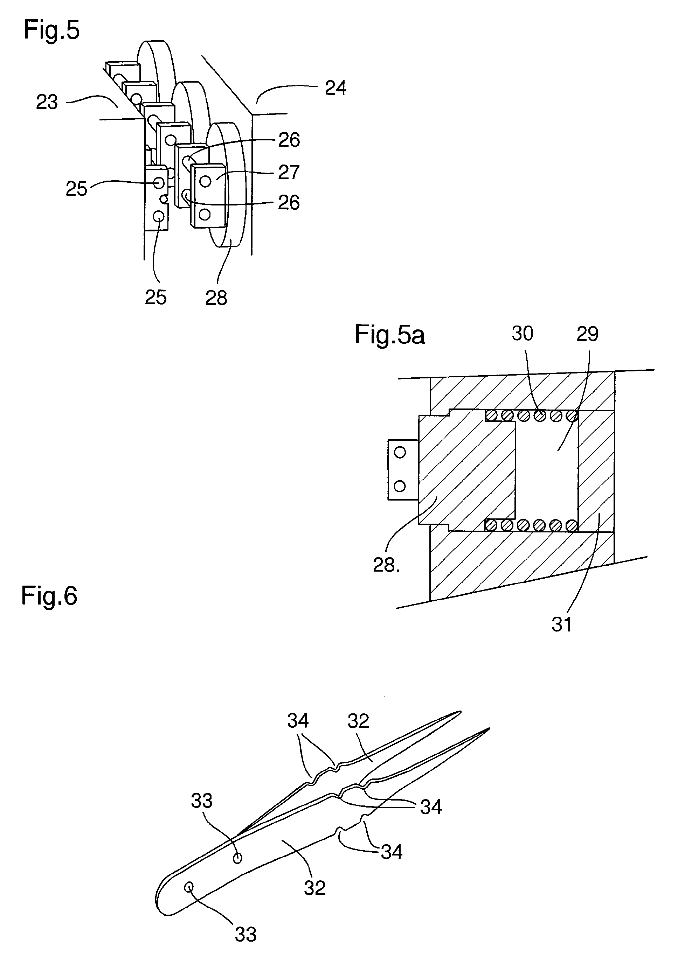

Le posage illustré aux figures 5 et 5a comporte un bâti comportant deux blocs 23, 24 se faisant face. Le premier bloc 23 comporte des organes de maintien fixes formés ici par deux goupilles 25 parallèles entre elles et par rapport à la face du bloc 23 sur lequel elles sont fixées. Le second bloc 24 comporte des organes de maintien mobiles 26 formés également par des goupilles parallèles entre elles et par rapport à la face du bloc 24 sur lequel elles sont montées. Ces goupilles 26 sont fixées entre deux entretoises 27 solidaires d'un noyau 28 monté coulissant dans un alésage 29 du bloc 24. Ce noyau 28 est soumis à l'action d'un ressort 30 prenant appui sur un bouchon 31 vissé dans le bloc 24. En position avancée le noyau 28 bute contre un épaulement de l'alésage 29.

Ce posage ou magasin est destiné à maintenir des brucelles du type de celles illustrées à la figure 6. Ces brucelles comportent deux branches 32 fixées ensemble à l'une de leurs extrémités. Cette extrémité comporte deux perçages 33 permettant la fixation de la brucelle dans le dispositif d'indexation du préhenseur par les deux billes 12.

Dans la partie médiane des branches 32, chacune de celles-ci comporte des entailles semi-circulaires 34 débouchant sur leurs tranches opposées. Deux entailles 34 par tranche de chaque branche 32 sont prévues, dont l'entraxe correspond à celui des goupilles 25 et 26 du magasin.

Pour placer les brucelles dans le magasin la brucelle est orientée dans un plan parallèle aux faces des blocs 23, 24 portant les goupilles 25, 26 puis les pointes de la brucelle sont introduites entre lesdites goupilles 25, 26 jusqu'au moment où les entailles 34 sont en face des goupilles. La brucelle est ensuite tournée d'un quart de tour et les goupilles 25, 26 s'engagent dans les entailles 32. Le noyau 28 recule puis avance pour maintenir les goupilles 26 appliquées contre les tranches des branches de la brucelle.

La brucelle peut alors être prise par un robot comportant le préhenseur. Le préhenseur est amené de telle sorte que la partie arrière de la brucelle soit introduite dans le dispositif d'indexation et que les billes 12 coopèrent avec les perçages 33 de la brucelle. Une rotation d'un quart de tour de la brucelle, donc du préhenseur, libère les goupilles 25, 26 des entailles 34 et la brucelle peut être extraite du magasin.

Il faut noter que lorsque les entailles 34 de la brucelle sont engagées sur les goupilles 25, 26 du magasin le retrait du préhenseur provoque le désaccouplement de la brucelle du dispositif d'indexation en vainquant la force d'appui des billes 12.

Le robot muni d'un préhenseur peut ainsi aller chercher dans le magasin la brucelle désirée pour une opération donnée puis l'y replacer.

La forme d'exécution illustrée est uniquement un exemple particulier de réalisation qui peut être modifié sans sortir du cadre de l'invention revendiquée.

Dans une variante les brucelles peuvent comporter deux branches asymétriques. L'une des branches est plane et épaisse, pratiquement non déformable, tandis que l'autres est mince et flexible, déformable élastiquement.

Ce qui reste constant dans l'invention, c'est que la brucelle comporte des formations ou moyens de fixation coopérant avec des moyens correspondants du dispositif de fixation permettant de fixer de façon amovible et dans une position déterminée la brucelle sur le préhenseur.

Par ailleurs, les brucelles peuvent également comporter des formations destinées à coopérer avec des formations correspondantes d'un magasin ou posage dans lequel ces brucelles peuvent être entreposées dans l'attente d'être accouplées au préhenseur.

## Revendications

1. Préhenseur pour brucelles, **caractérisé par le fait qu'**il comporte un boîtier (1), destiné à être monté sur le bras d'un robot, renfermant d'une part un dispositif de fixation et d'indexation (6-14) d'une brucelle (3) et d'autre part un dispositif d'actionnement des branches de la brucelle comportant un organe moteur (4,17) actionnant des poussoirs (21) agissant sur les branches de ladite brucelle.

2. Préhenseur selon la revendication 1, **caractérisé par le fait que** le boîtier (1) est ouvert à l'une de ses extrémités pour permettre l'introduction d'une brucelle (3) dans le dispositif de fixation et d'indexation (6-14).

3. Préhenseur selon la revendication 1 ou la revendication 2, **caractérisé par le fait que** le dispositif de fixation et d'indexation (6-14) comporte des moyens (9) de positionnement de la partie arrière d'une brucelle par rapport au boîtier (1) et des moyens de fixation temporaire (10,12,13) coopérant avec des moyens correspondants (33) de la brucelle pour maintenir celle-ci, avec une force déterminée dans le dispositif de fixation.

4. Préhenseur selon l'une des revendications précédentes, **caractérisé par le fait que** lorsqu'une brucelle est fixée et positionnée dans le dispositif de fixation et d'indexage, elle s'étend substantiellement suivant l'axe longitudinal du préhenseur.

5. Préhenseur selon l'une des revendications précédentes, **caractérisé par le fait que** l'accouplement et le désaccouplement d'une brucelle dans le préhenseur s'effectuent par un mouvement relatif axial de ces éléments.

6. Préhenseur selon l'une des revendications précédentes, **caractérisé par le fait que** les poussoirs (28) sont constitués par l'extrémité libre des bras (20) d'une pince, ces bras (20) étant fixés sur un écrou (19) en prise avec une vis (17) entraînée en rotation par un moteur rotatif (4).

7. Préhenseur selon l'une des revendications précédentes, **caractérisé par le fait qu'**il comporte un circuit de commande de l'organe moteur (4) piloté par un circuit de mesure détectant la force exercée sur les branches de la brucelle (3) par les poussoirs (21).

8. Brucelle destinée à être fixée de façon amovible sur le préhenseur de la revendication 1, **caractérisée par le fait que** la partie arrière de la brucelle comporte des formations d'accouplement destinées à coopérer avec les moyens correspondants du dispositif de fixation et d'indexage du préhenseur.

9. Brucelle selon la revendication 8, **caractérisée par le fait qu'**elle comporte une branche épaisse rigide et une seconde branche élastiquement déformable.

10. Brucelle selon la revendication 8 ou la revendication 9, **caractérisée par le fait qu'**elle comporte encore des formations de fixation coopérant avec des formations correspondantes d'un posage destiné au rangement des brucelles.
